# FASCICULE DE BREVET EUROPEEN

(11) **EP 2 043 587 B1**
(45) Date de publication et mention de la délivrance du brevet: **12.02.2014**
(21) Numéro de dépôt: 07823269.1
(22) Date de dépôt: 09.07.2007
(51) Int. Cl.: A61K 6/06, A61L 24/00, C04B 28/34

(54) **NOUVEAU COMPOSE PHOSPHO-CALCO-STRONTIQUE ET SES UTILISATIONS**
NEUARTIGE PHOSPHOR-CALCIUM-STRONTIUM-VERBINDUNGEN UND VERWENDUNGEN DAVON
NOVEL PHOSPHORUS-CALCIUM-STRONTIUM COMPOUND AND USES THEREOF

(30) Priorité: 12.07.2006 FR 0606372
(43) Date de publication de la demande: 08.04.2009
(73) Titulaire: Centre National de la Recherche Scientifique, 75016 Paris (FR); Université de Montpellier I, 34967 Montpellier Cedex 2 (FR)
(72) Inventeur: BOUDEVILLE, Philippe, F-34830 Clapiers (FR); MICHAILESCO, Pierre, F-34740 Vendargues (FR); CASTANY, Emmanuel, F-34750 Villeneuve-les-Maguelone (FR); VERT, Michel, René, F-34170 Castelnau-le-Lez (FR)
(74) Mandataire: Corizzi, Valérie
(86) Numéro de dépôt international: PCT/FR2007/001176
(87) Numéro de publication internationale: WO 2008/006970

(56) Documents cités:
- WO-A-03/103734
- WO-A2-2005/115488
- US-B1- 6 593 394
- DATABASE WPI Week 200532 Derwent Publications Ltd., London, GB; AN 2005-306850 XP002419537 & CN 1 559 888 A (UNIV XI-AN COMMUNICATION) 5 janvier 2005 (2005-01-05) cité dans la demande
- KANDORI KAZUHIKO ; SAITO MIWA ; SAITO HIROSHI ; YASUKAWA AKEMI ; ISHIKAWA TATSUO: "Adsorption of protein on non-stoichiometric calcium-strontium hydroxyapatite" COLLOIDS AND SURFACES A: PHYSIOCHEMICAL AND ENGINEERING ASPECTS, vol. 94, no. 2-3, 1995, pages 225-230, XP002419876
- CHABCHOUB S., DOGGUY M.: "Etude des systemes CaHPO4-MHPO4-H2O (M=Sr, Ba) a 50°C", J. OF TH. ANALYSIS, vol. 45, no. 3, 1 September 1995 (1995-09-01), pages 519-534,
- Chabchoub ET AL: "etude des systemes Cahpo4-mhpoa-h2o", journal of thermal analysis, 1 September 1995 (1995-09-01), pages 519-534, XP055075050, ISBN: 15728943 Retrieved from the Internet: URL:http://rd.springer.com/content/pdf/10. 1007/BF02548784.pdf [retrieved on 2013-08-13]

## Description

La présente invention a pour objet un nouveau composé phospho-calcostrontique, un procédé pour sa préparation et son utilisation comme ciment endodontique.

Elle a également pour objet les compositions de ciment endodontique comprenant ce nouveau composé.

L'endodontie est une discipline de l'odontologie qui concerne le traitement des maladies de la pulpe dentaire (pulpite, abcès dentaire...).

La pulpite est la rage de dent classique : douleur vive permanente augmentée par le chaud et le froid (inflammation et hyperémie pulpaire après traumatisme ou carie). La gangrène pulpaire intervient lorsque l'inflammation pulpaire n'est pas traitée ; les bactéries pénètrent dans la dent et nécrosent la pulpe. Les douleurs provoquées (chaud, froid, pression) et les crises sont plus fortes. Lorsque la nécrose pulpaire n'est pas soignée, les bactéries gagnent l'os alvéolaire environnant et le détruisent (lésion péri-apicale) en provoquant un kyste ou un granulome. Il faut alors dépulper la dent. Cette lésion guérit généralement suite à la désinfection et l'obturation des racines, dans certains cas par chirurgie endodontique.

Le traitement canalaire ou endodontique est une technique visant à extirper le tissu pulpaire à l'intérieur des canaux ou des racines d'une dent, à mettre en forme, désinfecter et sceller le ou les canaux et d'une manière générale l'arbre canalaire, c'est-à-dire l'obtention d'une obturation tridimensionnelle.

Après exérèse de la pulpe, on procède à la mise en forme mécanique du canal radiculaire et à sa désinfection chimique par irrigation répétée du canal avec NaClO (à 2,5% chlore actif) et H₂O₂ puis EDTA (à 5%) afin d'éliminer le maximum de débris pulpaires, de bactéries et de boue dentinaire (smear layer).
- si la pulpe est peu enflammée et si la radio ne présente pas de trace d'une lésion péri-apicale, la préparation mécanique et la désinfection chimique suffisent généralement pour une bonne asepsie et on procède au comblement du canal
   - le plus souvent par introduction à l'aide d'un lentulo ou bourre-pâte (vis sans fin) d'un ciment à base d'oxyde de zinc et d'eugénol (ZOE), avec éventuellement un maître cône de gutta-percha qui permet de compacter la pâte de ciment avant prise dans le canal et les canaux secondaires adjacents, mais surtout facilite une désobturation ultérieure éventuelle en cas de nécessité de retraitement ou pour placer un tenon faux moignon devant soutenir une prothèse fixée.
   - plus rarement, afin d'obtenir une meilleure étanchéité du comblement, le praticien peut avoir recours à une technique compressive à la gutta-percha (condensation latérale à froid, verticale à chaud ou thermomécanique). Elles sont considérées comme les techniques endodontiques de référence et enseignées en tant que telles dans les UFR d'Odontologie en France. Ces techniques ont l'inconvénient d'être longues et de nécessiter une bonne technicité du praticien.
- s'il y a nécrose pulpaire profonde et/ou lésion péri-apicale, après exérèse pulpaire et préparation, on traite d'abord le canal par de l'hydroxyde de calcium laissé en place un certain temps et éventuellement renouvelé avant d'effectuer le comblement comme ci-dessus.

Dans certains cas plus complexes, le praticien peut être amener à placer avant le comblement un bouchon apical (apical plug) ou à pratiquer une obturation *a retro* de l'apex ou encore à combler une perforation pathologique ou accidentelle de la paroi du canal. Le matériau de choix actuel est le MTA (Mineral Tri Oxyde Aggregate), un ciment à base de silicate d'aluminium et de calcium.

Après comblement du canal, la partie coronaire est obturée soit par un matériaux provisoire (Cavit®... IRM®...) si une réintervention est prévue dans un avenir proche, soit par un matériau définitif (amalgame, ciment verre ionomère, compomère, composite...) ou bien par mise en place d'une prothèse fixée.

Les principaux matériaux utilisés en endodontie sont l'hydroxyde de calcium, les ciments à l'oxyde de zinc-engénol, l'oxyde de calcium lourd, le MTA, la gutta-percha :
- L'hydroxyde de calcium Ca(OH)₂:
   L'utilisation de l'hydroxyde de calcium en endodontie a été introduite en 1920 par Hermann (Hermann BW. Calciumhydroxyd als mittle zum behandel und fullen von zahnwurzelkanalen. Wurzburg. Med Diss V 29, 1920).

Quatre qualités sont aujourd'hui à peu près unanimement reconnues à l'hydroxyde de calcium (Tronstad L. Endodontie clinique. Traduction française de Laudenbach P, Medecine-Sciences Flammarion, Paris 1993, Chap. 5): action antimicrobienne, action anti-inflammatoire par résolution des exsudats qui entretiennent l'inflammation, l'inhibition de la résorption du tissu dentaire et l'induction de la formation de tissus durs.

L'hydroxyde de calcium sert également de tampon chimique vis-à-vis des ciments de restauration généralement acides et de tampon thermique vis-à-vis des restaurations métalliques. Il n'inhibe pas la polymérisation des résines et composites.

L'hydroxyde de calcium présente toutefois un certain nombre d'inconvénients :
- utilisé seul ou en pâte, il ne durcit pas, se rétracte au séchage et, ayant une solubilité non nulle bien que faible, il disparaît assez rapidement. Il ne permet donc pas d'obtenir un comblement étanche et n'est utilisé que comme matériau temporaire pour la désinfection du canal et le traitement des lésions péri-apicales.
- sa radio-opacité est égale à celle de la dentine, il faut donc lui ajouter un produit de contraste
- lorsqu'on l'utilise en association avec une base résine pour obtenir son durcissement, celle-ci est en général hydrophobe et limite la diffusion des ions hydroxyle et calcium, d'où un effet antimicrobien quasi négligeable.
- Ciments à l'oxyde de zinc - eugénol (ZOE, zinc oxide-eugenol cement) :
   L'eugénol ou 4-allyl-2-methoxyphénol constitue 96% en poids de l'essence de girofle. Il réagit par sa fonction phénol avec l'oxyde de zinc ZnO pour donner un eugénolate de zinc.

Les propriétés biologiques des ciments ZOE sont essentiellement dues à la présence d'eugénol. Il a un effet sédatif de la douleur lors de pulpites. Il présente une activité antimicrobienne moyenne. Enfin, son introduction dans les canaux radiculaires est aisée à l'aide d'un lentulo.

Les ciments ZOE sont principalement utilisés en fond de cavité, pour le coiffage pulpaire et l'obturation endocanalaire.

Les ciments ZOE ont également un certain nombre d'inconvénients. Une odeur caractéristique de clou de girofle, un léger retrait à la prise, d'où la nécessité de recourir à des cônes de gutta-percha pour améliorer l'étanchéité, une solubilité non nulle et un effet anti-microbien fugace. Les ciments ZOE n'offrent pas de protection sur le long terme.
- L'oxyde de calcium « lourd », connu sous la marque Biocalex®, est produit à base d'oxyde de calcium CaO « lourd », précurseur de l'hydroxyde de calcium par hydratation avec un fort coefficient d'expansion, ce qui lui confère un effet antimicrobien et procure une bonne étanchéité des comblements. Toutefois son utilisation n'est pas dépourvue d'inconvénients : risque important d'éclatement de la dent, faibles caractéristiques mécaniques.
- Le MTA (Mineral Trioxide Aggregate) :
   Le MTA est un ciment de type portland à base de silicate d'aluminium et de calcium. La présence d'oxyde de calcium se traduit par une certaine alcalinité lors de la prise, favorable à une activité antimicrobienne et à l'induction du processus de dentinogénèse. Il procure une bonne étanchéité. Ses indications sont : le coiffage pulpaire, l'apexification, la création d'un bouchon apical, la réparation de perforations et l'obturation *a retro.* Il n'est cependant pas utilisé pour le comblement complet d'un canal en raison de son manque de fluidité et de sa trop grande résistance, une fois pris, qui ne permet pas la désobturation.
- La gutta-percha :
   La gutta-percha, ou trans 1,4 poly-isoprène, est un polymère naturel extrait d'un arbre, le Palaquium Gutta-Bail. Elle est incompressible mais thermoplastique au-delà de 60°C ce qui lui permet de prendre la forme de l'endodonte. Elle se rigidifie au refroidissement.

Elle est toujours utilisée en association avec un feuil de ciment (généralement ciment ZOE à prise lente). Bien que les techniques compressives soient considérées comme les techniques de référence, elles sont rarement pratiquées en routine car elles nécessitent un temps d'intervention beaucoup plus long incompatible avec la nomenclature actuelle des actes professionnels. D'autre part elles requièrent une bonne technicité du praticien nécessitant une formation spécifique.

La littérature a cité à plusieurs reprises les ciments phosphocalciques en mentionnant la possibilité d'un usage en endodontie (US-4,518,430 et US-5,997,624). Toutefois, l'essentiel des publications et des applications relatives à ce biomatériau concerne l'orthopédie.

La formule du premier ciment au phosphate de calcium à usage dentaire, à base d'oxyde de calcium et d'acide orthophosphorique, a été publiée en 1832. Ce produit a toutefois très vite été supplanté par les ciments au phosphate de zinc, beaucoup plus résistants sur le plan mécanique et vis-à-vis de l'érosion chimique.

Les ciments hydrauliques phosphocalciques à usages orthopédiques sont obtenus par mélange d'une poudre contenant un phosphate de calcium à caractère acide et un phosphate de calcium à caractère basique avec de l'eau ou une solution aqueuse tamponnée. Les composées solides se dissolvent dans la phase aqueuse et les ions calcium et phosphate ainsi libérés vont reprécipiter sous forme d'un phosphate de calcium de basicité intermédiaire moins soluble que les produits de départ : hydrogénophosphate de calcium dihydraté (brushite ou DCPD) ou hydroxyapatite stoechiométrique (HA) ou déficiente en calcium (CDHA). C'est l'enchevêtrement des cristaux précipités qui assurera les caractéristiques mécaniques du ciment. Par exemple :

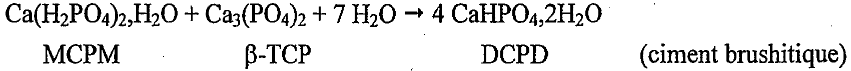

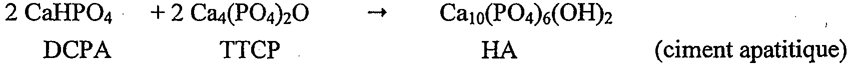

Les ciments à base d'oxyde de calcium et d'hydrogénophosphate de calcium ou de dihydrogénophosphate de calcium, lorsqu'ils sont mélangés avec de l'eau en vue de leur application, donnent de l'hydroxyapatite et éventuellement de l'hydroxyde de calcium (en fonction de la quantité de CaO mise en oeuvre).

Avec un excès d'oxyde de calcium, ces ciments sont trop basiques pour un usage en orthopédie comme matériau de substitution osseuse, mais ils sont parfaitement adaptés pour des traitements comblements en endodontie.

Ce type de ciment a une activité antimicrobienne satisfaisante dès qu'un rapport Ca/P = 2 est atteint et une activité antimicrobienne comparable à celle de l'hydroxyde de calcium pur dès qu'un rapport Ca/P = 2,5 est atteint. Toutefois, ces ciments présentent plusieurs défauts qui rendent leur utilisation dans le traitement endodontique difficilement envisageable : ils sont difficiles à manipuler comparativement aux ciments ZOE, et ils ne sont pas radio-opaques.

La radio-opacité d'un ciment de comblement canalaire est nécessaire pour deux raisons. Elle permet de vérifier le bon remplissage du canal et de savoir rapidement si une dent a déjà été traitée ou non au cas où le patient n'est pas un patient habituel. La radio-opacité d'un ciment de comblement canalaire est d'ailleurs obligatoire pour une prise en charge du traitement par les assurances maladies. Or ce type de ciment, ayant une composition identique à celle de la dentine et de l'émail, a une radio-opacité équivalente et non supérieure.

L'adjonction, dans la formule du ciment, d'un composé radio-opaque tel qu'un sel de Baryum est envisageable, mais elle aurait pour conséquence une dégradation des propriétés mécaniques et antimicrobiennes du ciment.

La manipulation du ciment par le praticien doit être aisée pour ne pas le rebuter malgré les qualités du produit. La préparation des ciments à base d'oxyde de calcium et d'hydrogénophosphate de calcium (mélange de la poudre au liquide) est simple et identique à celle des ciments au phosphate de zinc et ZOE. Par contre, leur tenue sur le lentulo n'est pas parfaite et si l'introduction du ciment dans des dents extraites est réalisable (test de laboratoire ex vivo), celle-ci sera beaucoup plus délicate sur un patient en cabinet.

L'invention a donc pour fondement la mise au point d'un ciment destiné à un usage endodontique, notamment au comblement des canaux radiculaires des dents dépulpées, ce ciment ayant de bonnes propriétés mécaniques, des propriétés antimicrobiennes durables, une rhéologie permettant sa mise en place dans de bonnes conditions. En outre on a mis au point un ciment qui est radio-opaque et expansif lorsqu'il prend en masse et qui ainsi assume un comblement étanche du canal à obturer, complétant le panel des propriétés positives du ciment.

La mise au point de ce ciment a reposé sur un procédé de production d'un nouveau composé phospho-calco-strontique qui répond à la formule (I) ci-dessous :

Ca₍₁₋ₓ₎Sr₍ₓ₎HPO₄ (I)

dans laquelle x représente un nombre compris entre 0,2 et 0,8, de préférence entre 0,3 et 0,7, encore plus préférentiellement entre 0,4 et 0,6, et avantageusement entre 0,45 et 0,55.

Le document Chabchoub S. et al., Journal of Thermal Analysis, 1995, 45(3), 519-534, étudie la possibilité de formation de solutions solides Ca₍₁₋ₓ₎SrₓHPO₄ par substitution du calcium par du strontium dans l'hydrogénophosphate de calcium et inversement dans le système CaHPO₄-SrHPO₄-H₂O par l'établissement du diagramme de solubilité dans l'eau du mélange ternaire (1-x) CaCO₃ + x SrCO₃ + H₃PO₄. Ils constatent que Ca remplace Sr pour donner une solution solide gardant la structure de l'α-SrHPO4 avec une diminution des paramètres de maille pour 0,6 < x < 1 et que Sr remplace Ca pour donner une solution solide gardant la structure de CaHPO₄ avec une augmentation des paramètres de maille pour 0,05 < x < 0,25. Enfin pour 0,25 < x < 0,6, le mélange est biphasé et contient des proportions variables de Ca_{0,75}Sr_{0,25}HPO₄ et de Ca_{0,4}Sr_{0,6}HPO₄. Toutefois, leur préparation est faite par des méthodes de précipitation en solution avec atteinte d'un équilibre, dont la position est fonction de différents paramètres expérimentaux et qui, en conséquence, ne peuvent pas être employées à l'échelle industrielle ou tout simplement pour obtenir de façon reproductible un composé choisi. Ce document ne mentionne nullement l'utilisation d'un tel composé pour préparer un ciment dentaire.

Le document Kikuchi M. et al., Journal of Solid State Chemistry, 1994, 113(2), 373-378, concerne également l'analyse de cristaux d'hydroxyapatite substituée par du strontium sans qu'une méthode de production reproductible ou industrialisable y soit décrite pour un composé choisi. Ce composé est caractérisé par un rapport (Ca+Sr)/P = 1,67, différent de celui de l'invention (Ca+Sr)/P = 1,0 et ne pourrait en aucun cas s'y substituer.

Le composé de formule (I) avec x compris entre 0,45 et 0,55 est nouveau et constitue un premier objet de l'invention. Ce composé, qui garde la structure de CaHP04, se situe dans un domaine où selon Chabchoub S. *et al* on obtient un mélange biphasé (vide supra). Il peut être obtenu grâce à sa méthode de synthèse particulière qui permet de travailler hors des équilibres thermodynamiques classiques et cinétiques de dissolution-précipitation en solution aqueuse.

Le composé de formule (I) est un hydrogénophosphate mixte de strontium et de calcium anhydre. Selon l'invention, ce composé est préparé par mécanosynthèse à partir de bis-dihydrogénophosphate de calcium monohydraté (ou MCPM, mono calcium phosphate monohydrate) et d'hydroxyde de strontium octahydraté, et éventuellement d'acide ortho phosphorique, ou d'hydroxyde de calcium, c'est-à-dire qu'il comporte une étape de mélange des produits de départ sous forme solide, suivant la réaction :
Pour 0,5 ≤ x ≤ 0,8

   (1-x) Ca(H₂PO₄)₂,H₂O + (2x-1) H₃PO₄ + x Sr(OH)₂,8H₂O → Ca₍₁₋ₓ) Srₓ HPO₄ + (9x +1) H₂O
Pour 0,2 ≤ x ≤ 0,5

   Ca(H₂PO₄)₂,H₂O + 2x Sr(OH)₂,8H₂O + (1-2x) Ca(OH)₂ → 2 Ca₍₁₋ₓ₎ Srₓ HPO₄ + (16x +3) H₂O

Notamment dans le cas où x = 0,5 :

Ca(H₂PO₄)₂,H₂O + Sr(OH)₂,8H₂O → 2 Ca_{0,5} Sr_{0,5} HPO₄ + 11H₂O

Le mélange des produits de départ de la réaction peut être fait dans un broyeur à billes ou par simple mélange dans un mortier. En pratique tout moyen d'agitation mécanique des poudres convient à la préparation du composé de formule (I). On peut introduire une petite quantité d'eau (contenant éventuellement l'acide orthophosphorique H₃PO₄ ou l'hydroxyde de calcium) dans le mélangeur pour faciliter le mélange des poudres.

La réaction se fait à température ambiante, elle peut nécessiter un temps de mélange allant de quelques minutes à une heure. Ensuite le produit est séché par tout moyen connu de l'homme du métier, comme par exemple le passage dans un four ou une étuve ou par atomisation. Le chauffage du produit de l'invention peut également être fait sous pression réduite de façon à faciliter l'évaporation de l'eau.

Le document Yokogawa Y. et al., Chemistry Letters, 1996, 45(4), 161-166, décrit un procédé mécano chimique par voie humide de préparation d'une apatite déficiente en calcium et en strontium à partir de CaHPO₄.2H₂O, de CaCO₃, SrHPO₄ et Sr(OH)₂.8H₂O. Toutefois, les conditions de ce procédé ne sont pas les plus favorables comme l'ont montré les inventeurs (Boudeville et al., Key Engineering. Materials 2004 ;254-256 :103-106) et ne peuvent conduire au composé de formule (I).

Le composé de formule (I) comprend du strontium. Celui-ci joue un rôle important dans le processus de minéralisation osseuse : il est présent naturellement dans la phase minérale des os, il remplace facilement le calcium dans l'os, il stimule l'activité des ostéoblastes tout en inhibant partiellement celle des ostéoclastes et il renforce la solidité de l'os. Pour ces raisons il est utilisé dans le traitement *per os* de l'ostéoporose post-ménopausique. De plus, il est plus radio-opaque que le calcium.

Dans le cas du traitement d'une dent par comblement du canal pulpaire, on peut s'attendre à ce que le strontium ait une activité stimulante sur l'activité des odontoblastes (cellules de la pulpe), et sur les cémentoblastes (cellules du cément, qui entoure et protège la dentine).

L'apport de strontium dans le composé de formule (I), et dans l'optique d'une utilisation pour la préparation d'un ciment endodontique, est triple :
(i) de masse atomique supérieure à celle du calcium, il rend le ciment plus radio-opaque ;
(ii) sous la forme utilisée, il participe à la réaction de prise, les apatites calciques et strontiques formant une solution solide continue, à la différence des sels de baryum classiquement utilisés pour augmenter la radio-opacité ;
(iii) stimulant l'activité des ostéoblastes, on peut s'attendre à ce qu'il stimule également l'activité des cémentoblastes pour la formation du bouchon (ou barrière) apical, la meilleure des garanties pour éviter le développement ou la reprise d'une lésion péri apicale.

On connaît dans l'art antérieur, notamment par WO 2005/115488, des ciments utilisés comme substituts osseux, à base d'hydrogénophosphate de calcium dihydraté (CaHPO₄,2H₂O), d'oxyde de calcium (CaO) et de carbonate de strontium. Toutefois dans une telle composition de ciment le strontium ne participe que partiellement à la réaction de prise, le sel de strontium est dispersé dans la phase hydroxyapatite de calcium et il en augmente les propriétés mécaniques.

Les ciments décrits dans ce document sont destinés à un usage orthopédique : substitut osseux injectable ou composite contenant des microsphères d'un polymère rapidement biodégradable afin d'y créer des macroporosités potentielles, tandis que le ciment de l'invention est à usage endodontique pour le traitement et le comblement de canaux radiculaires avec introduction au lentulo et se doit d'être microporeux.

Leurs compositions sont différentes :
*ciment orthopédique:*
   - phase solide : 6 CaHPO₄,2H₂O + 2,5 CaO + 1,5 SrCO₃ (60% m/m) + PLAGA (40% m/m) (PLAGA = microsphères de poly(DL-lactic acid-co-glycolic acid)
   - phase liquide : tampon phosphate d'ammonium pH 7, 0,75 M et rapport liquide/poudre = 0,5 ml g⁻¹

Dans le ciment de l'art antérieur, CaO est en défaut pour obtenir un ciment neutre, dans celui de l'invention, il y a un fort excès de CaO pour obtenir par son hydratation l'expansion nécessaire à l'étanchéité du comblement et une activité antimicrobienne rapide et à long terme ; le strontium y est également en plus grande quantité pour avoir une radio-opacité suffisante.

Un hydrogénophosphate mixte de calcium et de strontium anhydre est mentionné dans WO 2005/115488 sans qu'un mode de préparation industriel d'un tel composé soit décrit. Il n'est pas non plus décrit de mise en oeuvre d'un tel composé dans un ciment osseux.

Le document CN-1559888 décrit un ciment osseux à base d'hydrogénophosphate de calcium, d'hydrogénophosphate de strontium et d'oxodiphosphate de tétracalcium (ou tétracalcium phosphate, TTCP). Le composé de formule (I) n'est pas utilisé dans ce ciment qui résulte du mélange :
Phase solide : Ca₄(PO₄)₂O + CaHPO₄ + SrHPO₄
Phase liquide : solution aqueuse de H₃PO₄ à 0,5-1 mol/l.

Ce ciment a de très bonnes propriétés mécaniques, essentiellement dues au TTCP, qui sont appropriées pour une application à l'os mais qui ne permettraient pas une réintervention s'il était appliqué sur une dent. Sa rhéologie n'est pas adaptée à un comblement canalaire.

Ce ciment ne libère pas d'ions hydroxyle, il est donc dépourvu d'effet antimicrobien. Il ne produit pas d'expansion au cours de sa réaction de prise et donc ne présente pas les propriétés d'herméticité recherchées en application endodontique.

En outre, le produit de départ Ca₄(PO₄)₂O n'est pas disponible commercialement et est difficile à préparer.

Un autre objet de l'invention est une composition pour la préparation extemporanée d'un ciment, comprenant une phase solide (PS) et une phase liquide (PL) séparées. La phase solide comprend un mélange minéral de formule (II) :

6 Ca₍₁₋ₓ₎Sr₍ₓ₎HPO₄ + y CaO + z A (II)

dans laquelle :
x représente un nombre compris entre 0,2 et 0,8, comme dans la formule (I) ci-dessus et avec les mêmes variantes préférées ;
y représente un nombre compris entre 5 et 12 ;
A représente un composé radio-opacifiant ;
z représente un nombre compris entre 1 et 3.

A peut être un sel de bismuth, de baryum ou de strontium sous forme de carbonate, de sulfate, de fluorure ou de phosphate.

Lorsque l'application visée est l'obturation du canal endodontique, on préfère choisir y entre 7 et 12, de préférence entre 8 et 10 pour le ciment de formule (II).

Lorsque l'application visée est le coiffage pulpaire on préfère des compositions de ciment dans lesquelles y est compris entre 5 et 7. De préférence z est compris entre 1,5 et 2,5.

Le ciment de l'invention est biocompatible et injectable. Il est potentiellement biodégradable.

Le mélange de formule (II) est préparé par mélange sous forme de poudres de l'hydrogénophosphate mixte de calcium et de strontium de formule (I), de l'oxyde de calcium (CaO) et du composé radio-opaque (A) dans les proportions de la formule (II).

La phase liquide de la composition de ciment est constituée par de l'eau ou par une solution aqueuse saline de pH compris entre 5 et 9. De préférence on utilise une solution aqueuse saline de pH compris entre 6 et 8, de préférence entre 6,5 et 7,5. Avantageusement on utilise une solution aqueuse comprenant de 0,2 à 1,5 mol/l d'un mélange de NaH₂PO₄ et de Na₂HPO₄ (tampon NaP) ou une solution aqueuse comprenant de 0,2 à 1,5 mol/l d'un mélange de NaH₂PO₄ et de glycérophosphate de sodium hexahydraté (tampon NaGP), ou une solution aqueuse comprenant 0,2 à 1,5 mol/l d'un mélange de NH₄H₂PO₄ et de (NH₄)₂HPO₄ (tampon NH₄P) ou encore une solution aqueuse comprenant de 0,2 à 1,5 mol/l de citrate de trisodium (tampon Cit).

Avantageusement, la composition de ciment de l'invention comprend en outre au moins un polymère. L'une ou l'autre de la phase solide (PS) ou la phase liquide (PL) de la composition de ciment de l'invention peut comprendre ce ou ces polymère(s).

Le polymère peut être un alginate, du chitosane, du dextran, du sulfate de dextran ou un polyacrylate. Avantageusement, le polymère est choisi parmi les polyacrylates, comme un polyacrylate de sodium et les alginates comme un alginate de sodium. De préférence on choisit un polyacrylate de poids moléculaire compris entre 2 000 et 50 000, avantageusement entre 3000 et 30 000, encore plus avantageusement entre 5000 et 20 000. De tels polymères sont disponibles commercialement.

Selon l'invention, lorsque le polymère est ajouté dans la phase solide, il représente de 1 à 5% en poids par rapport au poids total de la phase solide de la composition de ciment, avantageusement de 2 à 4%. Si le polymère est ajouté dans la phase liquide il représente de 0,2 à 10% en poids par rapport au volume total de la phase liquide. La quantité de polymère à employer dans la composition varie dans ces intervalles en fonction de la nature du polymère.

De préférence, la phase liquide comporte en outre une petite quantité d'un antimicrobien classique tel que NaClO.

Le rapport entre le volume de la phase liquide (L) et le poids de la phase solide (P) lors de la préparation de la composition de ciment est 0,2<L/P<0,7 ml/g, avantageusement entre 0,3<L/P<0,6 ml/g. Lorsque l'application visée est le comblement radiculaire, on préfère 0,4<L/P<0,5 ml/g, lorsque l'application est le coiffage pulpaire, on préfère 0,35<L/P<0,4 ml/g.

Lorsque l'on mélange la phase solide avec la phase liquide, l'hydrogénophosphate mixte de calcium et de strontium réagit avec l'oxyde de calcium suivant le schéma ci-dessous :

6Ca₍₁₋ₓ₎SrₓHPO₄+yCaO → Ca₍₁₀₋₆ₓ₎Sr₆ₓ(PO₄)₆(OH)₂ + (y-4) Ca(OH)₂

et l'hydroxyapatite précipite dans un bref délai (quelques minutes) après la mise en contact avec la phase liquide.

Selon une variante de l'invention, le composé 6Ca₍₁₋ₓ₎SrₓHPO₄ peut être remplacé dans cette étape par son précurseur, qui est un mélange de CaHPO₄, 2H₂O, ou de CaHPO₄, et de SrHPO₄.

En effet, 6Ca₍₁₋ₓ₎SrₓHPO₄ = 6(1-x)CaHPO₄(2H₂O)+6xSrHPO₄

En remplaçant dans la formulation du ciment (II) le composé (I) par un mélange de CaHPO₄, 2H₂O, ou de CaHPO₄, et de SrHPO₄. selon l'équation ci-dessus, on obtient également des ciments utilisables en endodontie. Lorsque l'on utilise ce mélange en remplacement du composé (I) dans la formulation (II), on obtient après mélange avec la phase liquide et réaction de prise, le même produit final.

Un autre objet de l'invention est donc une composition pour la préparation extemporanée d'un ciment, comprenant une phase solide (PS) et une phase liquide (PL) séparées, la phase solide comprenant un mélange minéral de formule (IIbis) :

6(1-x)CaHPO₄(2H₂O)+6xSrHPO₄+ y CaO + z A (IIbis)

dans laquelle x, y, z et A ont la même signification que ci-dessus dans la formule (II), les autres constituants, phase liquide et polymère, étant identiques à ceux décrits ci-dessus.

Un autre objet de l'invention est un procédé de préparation d'un ciment, à partir d'une composition comportant une phase solide et une phase liquide décrites ci-dessus et comportant au moins une étape dans laquelle on mélange la phase solide avec la phase liquide, étape dans laquelle se produit la réaction de formation d'une hydroxyapatite mixte de calcium et de strontium suivant le schéma ci-dessus.

Pendant toute la durée du stockage, le ciment est conservé sous forme de deux phases l'une solide, l'autre liquide, séparément. Les compositions de ciment de l'invention ont de bonnes propriétés de conservation dès lors que la phase solide est maintenue dans un emballage étanche à l'air et à l'humidité.

Un autre objet de l'invention est un kit dentaire comprenant une composition pour la préparation extemporanée d'un ciment telle que décrite ci-dessus sous forme d'une phase liquide et d'une phase solide conservées dans des compartiments séparés.

Un tel kit destiné au comblement des canaux radiculaires dentaires ou à une autre application dentaire peut être constitué de deux flacons distincts, de volumes adaptés à la consommation des cabinets dentaires.

On peut aussi prévoir des kits sous forme de flacons comportant chacun la quantité de phase solide et de phase liquide appropriées pour l'obturation d'une seule dent. Un tel conditionnement présente l'avantage d'éviter les erreurs de dosage au moment de la préparation du ciment et une éventuelle dégradation des qualités du ciment due à une ouverture répétée des flacons.

On peut également prévoir de conditionner la composition de ciment de l'invention dans un récipient à mélange direct extemporané tel qu'une carpule. On peut citer par exemple le dispositif de mélange décrit dans US-5,549,380 qui permet d'introduire la phase liquide dans la phase solide par aspiration au moment où le mélange doit être effectué. On peut également utiliser une poche souple à deux compartiments contenant chacun l'une des phases de la composition, les compartiments étant séparés par un opercule qui est brisé au moment où le mélange doit être effectué, permettant le passage de la phase liquide dans le compartiment contenant la phase solide.

La présence de strontium dans l'hydroxyapatite permet d'avoir un ciment radio-opaque sans dégradation des propriétés mécaniques et rhéologiques du ciment : temps de prise, résistance à la compression et injectabilité.

On observe en outre une libération régulière d'ions Sr²⁺ à partir du ciment pris, les ions Sr²⁺ étant susceptibles d'activer la reconstruction de la dentine par activation des odontoblastes.

Lors de la réaction de prise, le ciment de l'invention produit une légère expansion, suffisante pour assurer l'étanchéité de l'obturation et insuffisante pour occasionner une douleur ou fragiliser la dent.

Les compositions de ciment de l'invention ont également la propriété de libérer des ions OH- de façon régulière et prolongée, ce qui explique leur activité antibactérienne. Ces compositions sont d'une dureté suffisante pour leur utilisation dans le comblement d'un canal radiculaire. Toutefois elles sont d'une dureté légèrement inférieure à celle de la dentine, ce qui permet d'envisager leur retrait en cas de formation d'une nouvelle lésion péri apicale.

Les applications préférentielles de cette composition de ciment sont les suivantes :
- Obturation des canaux radiculaires dentaires postérieurement à une pulpectomie. Cette obturation est avantageusement opérée après avoir appliqué de façon répétée du dihydroxyde de calcium Ca(OH)₂ afin de faire se résorber la lésion péri apicale.
- Coiffage pulpaire (direct et indirect) : une adaptation de la composition et du rapport phase liquide/phase solide permet d'adapter la formule pour cet usage de façon à avoir un temps de prise plus court et une alcalinité moindre. Dans cette application, la composition de l'invention devrait favoriser la formation d'un pont dentaire cicatriciel.
- Réparation d'une perforation.
- Formation d'un bouchon apical.

Les compositions de ciment de l'invention présentent aussi l'avantage d'être compatibles avec la présence de résidus d'eau de javel dans le canal dentaire. En effet après pulpectomie, le dentiste nettoie le canal dentaire avec une solution d'hypochlorite de sodium, puis éventuellement d'ETDA ou de H₂O₂. Un parfait séchage des parois radiculaires est requis avant la mise en place des ciments classiques souvent non miscibles à une phase aqueuse avec risque de formation d'un hiatus. Ce n'est pas le cas du ciment de l'invention. Un rinçage insuffisant du canal dentaire ne gênera pas la prise de ce ciment.

### FIGURES:

Figure 1A : Radiologie d'une dent comblée avec un ciment ZOE
Figure 1B : Radiographie d'une dent comblée avec le ciment de l'exemple 4 (OP3)
Figure 2 : Inhibition de croissance d'un inoculum polymicrobien (test par diffusion en milieu gélosé solide) par le ciment OP3 et un ciment ZOE classique (a) fraîchement préparés et (b) préparés depuis 13 jours.
   ZIR : zone d'inhibition relative, c'est-à-dire comparée à l'inhibition par des échantillons d'hydroxyde de calcium pur fraîchement préparés (ZIR = 1 pour Ca(OH)₂).
Figure 3 : Quantité cumulée d'ions hydroxyle libérés au cours du temps à partir du ciment OP3 ; la droite horizontale représente la quantité maximale libérale.

### EXEMPLES:

### Exemple 1 : Préparation de l'hydrogéno phosphate mixte calcium-strontium Ca_{0,5}Sr_{0,5}HPO₄

Ce composé a été préparé par mécanosynthèse à partir de MCPM et d'hydroxyde de strontium octahydraté dans les conditions suivantes :

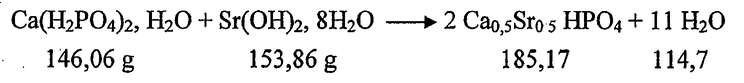

Broyage 20 min, broyeur planétaire Fritsch Pulverisette 6 excentricité 6 cm, bol porcelaine 500 ml, 4 billes porcelaine diamètre 30 mm, masse totale 248 g, surface totale 113 cm², vitesse de rotation 200 rpm.

Après 20 min de broyage, on obtient une masse pâteuse due à la présence d'eau résultant de la réaction (38,3%) la pâte est ensuite séchée 3 h à 120°C. Le gâteau est manuellement concassé et la poudre tamisée (tamis de 100 µm). On récupère ainsi environ 150-160 g de poudre tamisée soit un rendement de 80-83%.

### Exemple 2: Séchage de l'hydrogéno phosphate mixte calcium-strontium Ca_{0,5}Sr_{0,5}HPO₄ par atomisation (spray drying)

A 1200 g de pâte préparée en 4 fois comme dans l'exemple 1 et contenant 740 g de Ca_{0,5}Sr_{0,5}HPO₄ et 460 g d'eau, ont été ajoutés 1300 ml d'eau distillée (rapport masse de poudre sur masse de suspension de 30%). La suspension a été séchée par atomisation (Niro Atomizer Minor, température dans la cuve, 105°C et pression d'air 7 bar). On récupère 630 g de poudre sèche (rendement 85%) dont 80 % de la masse a une granulométrie inférieure à 50 µm.

### Exemple 3 : Préparation d'une composition de ciment endodontique avec du fluorure de strontium comme radio-opacifiant (noté OP3F)

### * phase solide

mélange 6 Ca_{0,5} Sr_{0,5} HPO₄ + 9 CaO + 2 SrF₂

### * phase liquide

solution tampon NaH₂PO₄ + Na₂HPO₄ 0,25 mole par litre en phosphate + citrate de sodium 0,25 mole par litre en citrate + polyacrylate de sodium (M = 5 100) pour 5% en masse/volume

### * rapport liquide sur poudre (L/P)

La poudre est mélangée à la phase liquide dans un rapport de 0,5 ml de liquide par gramme de poudre soit L/P = 0,5 ml g⁻¹. Lors des différents essais, les deux composants ont été mélangés sur une plaque de verre à la température ambiante de 27°C à l'aide d'une spatule. La résistance à la compression axiale est de 4 MPa, le temps de prise de 16 min et le temps de tenue correcte sur le bourre-pâte (lentulo) est de 10 min.

### Exemple 4 : Préparation d'une composition de ciment endodontique avec du carbonate de strontium comme radio-opacifiant (noté OP3)

### * phase solide

mélange 6 Ca_{0,5} Sr_{0,5} HPO₄ + 9 CaO + 2 SrCO₃ pour 97% en masse + polyacrylate de sodium (M = 20 000) pour 3% en masse

### * phase liquide

solution tampon NaH₂PO₄ + Na₂HPO₄, pH = 7, 0,5 mole par litre en phosphate + NaClO à 0,02% de Cl actif (l'hypochlorite de sodium n'est là qu'en tant qu'agent antimicrobien et antifongique pour éviter le développement d'une flore microbienne ou fongique dans le tampon au cours du temps après son ouverture).

### * rapport liquide sur poudre (L/P)

La poudre est mélangée à la phase liquide dans un rapport de 0,4 ml de liquide par gramme de poudre soit L/P = 0,4 ml g⁻¹. Lors des différents essais, les deux composants ont été mélangés sur une plaque de verre à 20°C ± 2°C à l'aide d'une spatule. Le mélange peut également se faire par broyage au pilon dans un mortier ou par agitation mécanique d'une capsule contenant la quantité voulue des deux phases (comme pour les amalgames ou les ciments au phosphate de zinc) ; technique testée à l'aide d'un mélangeur « capsule mixer HSM 1 » pendant 15 s.

### - Caractéristiques radiographiques:

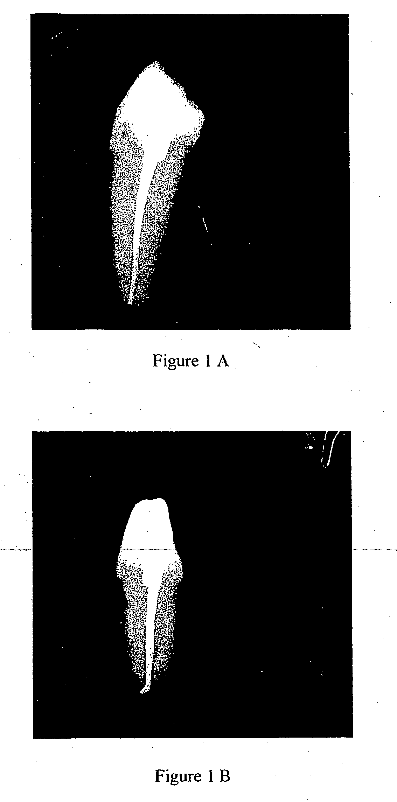

Comme on peut le constater sur les figures 1A et 1B, le ciment de l'invention possède une radio-opacité équivalente à celle de 3,2 mm d'aluminium, comparable à celle d'un ciment ZOE couramment utilisé.

### - Propriétés mécaniques :

Différentes compositions de ciment ont été testées pour leurs propriétés mécaniques, sur la base de la composition de l'exemple 4, en faisant varier le rapport P/L. Les résultats sont illustrés dans le tableau 1 ci-dessous :

| Ciments | Rapport L/P (ml.g-1) | Résistance à la compression axiale (MPa) | Consistance et manipulation (score : 0 à 5) | Temps de prise initiale 37°C(min) | Temps de prise finale à 37°C(min) | Expansion (en %) |
|---|---|---|---|---|---|---|
| OP3,NaPA 3% | 0,35 | 13 | 1 | 9 | 12 | 3,3 |
| OP3,NaPA 3% | 0,38 | 9,9 | 5 | 11 | 14 | 3,4 |
| OP3,NaPA 3% | 0,4 | 8,9 | 5 | 13 | 16 | 3,1 |
| OP3,NaPA 3% | 0,45 | 7,5 | 1 | 17 | 21 | 2,9 |

### -Propriétés microbiologiques :

L'activité antimicrobienne du ciment OP3 a été comparée à celle d'un ciment ZOE classique et à celle d'une pâte d'hydroxyde de calcium pur ; test d'inhibition de croissance d'un inoculum poly microbien prélevé sur de la plaque dentaire par diffusion en milieu gélosé solide. L'activité de ces deux ciments a été vérifiée sur des échantillons de ciment fraîchement préparés et des échantillons préparés depuis 13 jours. L'OP3 (RIZ = 1,26) et le ZOE (RIZ = 1,23) frais ont présenté une activité antimicrobienne supérieure à celle de l'hydroxyde de calcium (RIZ = 1 pour Ca(OH)₂) ; P ≈ 10⁻⁵, test ANOVA 1 facteur. Par contre, préparé depuis 13 jours, l'activité de l'OP3 reste équivalente à celle de l'hydroxyde de calcium frais (P = 0,07) alors que celle du ciment ZOE est quasiment nulle (7 des 8 échantillons n'ont pas présenté d'inhibition) comme on pouvait s'y attendre d'après la littérature.

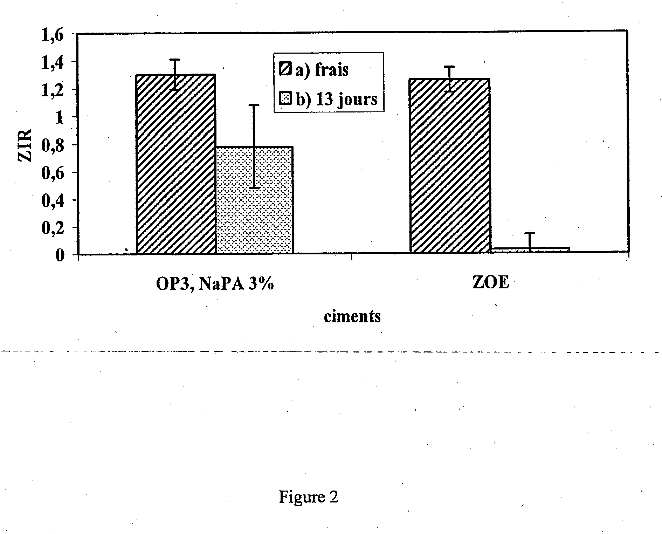

Ces résultats sont illustrés sur la figure 2.

### - Libération d'ions hydroxyle :

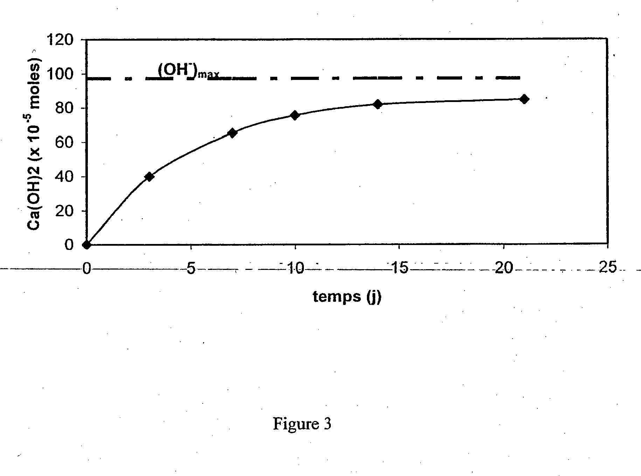

Des échantillons cylindriques de 0,5 g ont été immergés dans 10 ml d'eau distillée. Celle-ci a été renouvelée périodiquement et les ions hydroxyle libérés ont été dosés par pH-métrie (Figure 3).

La libération prolongée des ions hydroxyles explique l'activité antimicrobienne de ce ciment et leur diffusion dans des tubules dentinaires permet leur désinfection. Ceux-ci ne sont libérés qu'au contact d'une phase aqueuse qui, une fois le ciment introduit dans le canal radiculaire, est beaucoup moins importante par rapport à la masse du ciment que dans ce test *in vitro.* L'effet protecteur antimicrobien de ce ciment *in* vivo devrait donc être beaucoup plus long que dans ce test *in vitro* de 3 semaines.

### - Manipulabilité :

Lors de la préparation du ciment OP3, on constate l'obtention d'une consistance excellente, une bonne tenue sur le lentulo, une viscosité adaptée à l'utilisation clinique.

## Revendications

1. Composé répondant à la formule (I) ci-dessous :
Ca ₍₁₋ₓ₎Sr₍ₓ₎HPO₄ (I)
dans laquelle x représente un nombre compris entre 0,45 et 0,55.

2. Procédé de production d'un composé de formule (I) :
Ca ₍₁₋ₓ₎Sr₍ₓ₎HPO₄ (I)
dans laquelle x représente un nombre compris entre 0,2 et 0,8, **caractérisé en ce qu'**il comporte une étape de mélange de bis-dihydrogénophosphate de calcium monohydraté et d'hydroxyde de strontium octahydraté sous forme de poudre: suivant l'une des réactions :
Pour 0,5 ≤ x ≤ 0,8
(1-x) Ca(H₂PO₄)₂,H₂O + (2x-1) H₃PO₄ + x Sr(OH)₂,8H₂O → Ca₍₁₋ₓ₎ Srₓ HPO₄ + (9x +1) H₂O
Pour 0,2 ≤ x ≤ 0,5
Ca(H₂PO₄)₂,H₂O + 2x Sr(OH)₂,8H₂O + (1-2x) Ca(OH)₂ → 2 Ca₍₁₋ₓ₎ Srₓ HPO₄ + (16x +3) H₂O

3. Procédé selon la revendication 2, **caractérisé en ce que** le mélange est fait dans un broyeur à billes ou par simple mélange dans un mortier, en présence d'une petite quantité d'eau éventuellement en présence d'acide orthophosphorique ou d'hydroxyde de calcium, à température ambiante, suivi d'un séchage.

4. Composition comprenant une phase solide (PS) et une phase liquide (PL), la phase solide comprenant un mélange de minéraux de formule (II) :
6 Ca₍₁₋ₓ₎Sr₍ₓ₎HPO₄ + y CaO + z A (II)
dans laquelle :
x représente un nombre compris entre 0,2 et 0,8 ;
y représente un nombre compris entre 5 et 12 ;
A représente un composé radio-opaque ;
z représente un nombre compris entre 1 et 3, et
la phase liquide est constituée par de l'eau ou par une solution aqueuse saline de pH compris entre 5 et 9,
pour utilisation en tant que ciment endodontique.

5. Composition selon la revendication 4, dans laquelle x représente un nombre compris entre 0,3 et 0,7, encore plus préférentiellement entre 0,4 et 0,6, et avantageusement entre 0,45 et 0,55.

6. Composition selon la revendication 4 ou 5, dans laquelle A est choisi parmi un sel de bismuth, de baryum ou de strontium, d'un composé choisi parmi : un carbonate, un sulfate, un fluorure ou un phosphate.

7. Composition selon l'une quelconque des revendications 4 à 6, **caractérisée en ce que** y est choisi entre 7 et 12, et z est compris entre 1,5 et 2,5.

8. Composition selon l'une quelconque des revendications 4 à 7, **caractérisée en ce que** y est choisi entre 8 et 10.

9. Composition selon l'une quelconque des revendications 4 à 6, **caractérisée en ce que** y est choisi entre 5 et 7.

10. Composition selon l'une quelconque des revendications 4 à 9, **caractérisée en ce que** la phase liquide est choisie parmi :
- une solution aqueuse comprenant de 0,2 à 1,5 mol/l d'un mélange de NaH₂PO₄ et de Na₂HPO₄,
- une solution aqueuse comprenant de 0,2 à 1,5 mol/l d'un mélange de NaH₂PO₄ et de glycérophosphate de sodium hexahydraté,
- une solution aqueuse comprenant 0,2 à 1,5 mol/l d'un mélange de NH₄H₂PO₄ et de (NH₄)₂HPO₄.
- une solution aqueuse comprenant 0,2 à 1,5 mol/l de citrate de trisodium.

11. Composition selon l'une quelconque des revendications 4 à 10, **caractérisée en ce que** le rapport entre le volume de la phase liquide (L) et le poids de la phase solide (P) est 0,2 <L/P<0,7 ml/g, avantageusement 0,3 <L/P<0,6 ml/g.

12. Composition selon l'une quelconque des revendications 4 à 11, **caractérisée en ce qu'**elle comprend en outre au moins un polymère, choisi parmi les polyacrylates et les alginates.

13. Composition selon la revendication 12, **caractérisée en ce que** le polymère est un polyacrylate de poids moléculaire compris entre 2 000 et 50 000, avantageusement entre 3 000 et 30 000, encore plus avantageusement entre 5 000 et 20 000.

14. Composition selon l'une quelconque des revendications 12 ou 13, **caractérisée en ce que** le polymère est dans la phase solide et représente de 1 à 5% en poids par rapport au poids total de la phase solide, avantageusement de 2 à 4%.

15. Composition selon l'une quelconque des revendications 12 ou 13, **caractérisée en ce que** le polymère est dans la phase liquide, et représente de 0,2 à 10% en poids par rapport au volume total de la phase liquide.

16. Procédé de préparation d'un ciment à partir d'une composition selon l'une quelconque des revendications 4 à 15, **caractérisé en ce qu'**il comporte au moins une étape dans laquelle on mélange la phase solide avec la phase liquide et dans laquelle se produit la réaction :
6 Ca₍₁₋ₓ₎SrₓHPO₄+y CaO → Ca₍₁₀₋₆ₓ₎Sr₆ₓ(PO₄)₆(OH)₂ + (y-4) Ca(OH)₂

17. Un kit dentaire comprenant une composition selon l'une quelconque des revendications 4 à 15 sous forme d'une phase liquide et d'une phase solide conservées dans des compartiments séparés.

18. Utilisation d'une composition selon l'une quelconque des revendications 4 à 15 pour la préparation d'un ciment endodontique destiné à l'une des applications suivantes :
- l'obturation des canaux radiculaires dentaires,
- le coiffage pulpaire,
- la réparation d'une perforation,
- la formation d'un bouchon apical.

## Patentansprüche

1. Verbindung, die der folgenden Formel (I) entspricht:
Ca₍₁₋ₓ)Sr₍ₓ₎HPO₄ (I)
in der x eine Zahl zwischen 0,45 und 0,55 darstellt.

2. Verfahren zur Herstellung einer Verbindung der Formel (I):
Ca₍₁₋ₓ)Sr₍ₓ₎HPO₄ (I)
in der x eine Zahl zwischen 0,2 und 0,8 darstellt, **dadurch gekennzeichnet, dass** es eine Stufe des Mischens von Calcium-bis-dihydrogenphosphat-Monohydrat und Strontiumhydroxid-Octahydrat in Pulverform nach einer der Reaktionen umfasst:
Für 0,5 ≤ x ≤ 0,8
(1-x) Ca (H₂PO₄)₂, H₂O + (2x-1) H₃PO₄ + xSr (OH)₂, 8H₂O → Ca₍₁₋ₓ₎SrₓHPO₄ + (9x+1)H₂O
Für 0,2 ≤ x ≤ 0,5
Ca(H₂PO₄)₂, H₂O + 2xSr(OH)₂, 8H₂O + (1-2x)Ca(OH)₂ → Ca₍₁₋ₓ)SrₓHPO₄ + (16x+3)H₂O

3. Verfahren gemäß Anspruch 2, **dadurch gekennzeichnet, dass** das Mischen in einer Kugelmühle oder durch einfaches Mischen in einem Mörser in Gegenwart einer kleinen Wassermenge, gegebenenfalls in Gegenwart von Orthophosphorsäure oder Calciumhydroxid, bei Umgebungstemperatur durchgeführt wird, gefolgt von einer Trocknung.

4. Zusammensetzung, umfassend eine feste Phase (phase solide, PS) und eine flüssige Phase (phase liquide, PL), wobei die feste Phase ein Mineralgemisch der Formel (II) umfasst:
6Ca₍₁₋ₓ₎Sr₍ₓ₎HPO₄ + yCaO + zA (II)
in der:
x eine Zahl zwischen 0,2 und 0,8 darstellt;
y eine Zahl zwischen 5 und 12 darstellt;
A eine radioopake Verbindung darstellt;
z eine Zahl zwischen 1 und 3 darstellt und
die flüssige Phase durch Wasser oder eine wässrige Salzlösung mit einem pH zwischen 5 und 9 gebildet wird,
zur Verwendung als endodontischer Zement.

5. Zusammensetzung gemäß Anspruch 4, in der x eine Zahl zwischen 0,3 und 0,7, noch bevorzugter zwischen 0,4 und 0,6 und vorteilhafterweise zwischen 0,45 und 0,55, darstellt.

6. Zusammensetzung gemäß Anspruch 4 oder 5, in der A ausgewählt ist aus einem Salz von Bismut, Barium oder Strontium, einer Verbindung, ausgewählt aus einem Carbonat, einem Sulfat, einem Fluorid oder einem Phosphat.

7. Zusammensetzung gemäß einem der Ansprüche 4 bis 6, **dadurch gekennzeichnet, dass** y zwischen 7 und 12 ausgewählt ist und z zwischen 1,5 und 2,5 ausgewählt ist.

8. Zusammensetzung gemäß einem der Ansprüche 4 bis 7, **dadurch gekennzeichnet, dass** y zwischen 8 und 10 ausgewählt ist.

9. Zusammensetzung gemäß einem der Ansprüche 4 bis 6, **dadurch gekennzeichnet, dass** y zwischen 5 und 7 ausgewählt ist.

10. Zusammensetzung gemäß einem der Ansprüche 4 bis 9, **dadurch gekennzeichnet, dass** die flüssige Phase ausgewählt ist aus:
- einer wässrigen Lösung, die 0,2 bis 1,5 mol/l eines Gemisches aus NaH₂PO₄ und Na₂HPO₄ umfasst,
- einer wässrigen Lösung, die 0,2 bis 1,5 mol/l eines Gemisches aus NaH₂PO₄ und Natriumglycerophosphat-Hexahydrat umfasst,
- einer wässrigen Lösung, die 0,2 bis 1,5 mol/l eines Gemisches aus NaH₂PO₄ und (NH₄)₂HPO₄ umfasst,
- einer wässrigen Lösung, die 0,2 bis 1,5 mol/l Trinatriumcitrat umfasst.

11. Zusammensetzung gemäß einem der Ansprüche 4 bis 10, **dadurch gekennzeichnet, dass** das Verhältnis zwischen dem Volumen der flüssigen Phase (L) und dem Gewicht der festen Phase (P) 0,2 < L/P < 0,7 ml/g, vorteilhafterweise 0,3 < L/P < 0,6 ml/g ist.

12. Zusammensetzung gemäß einem der Ansprüche 4 bis 11, **dadurch gekennzeichnet, dass** sie außerdem wenigstens ein Polymer, ausgewählt aus Polyacrylaten und Alginaten, umfasst.

13. Zusammensetzung gemäß Anspruch 12, **dadurch gekennzeichnet, dass** das Polymer ein Polyacrylat mit einem Molekulargewicht von zwischen 2000 und 50000, vorteilhafterweise zwischen 3000 und 30000, noch vorteilhafter zwischen 5000 und 20000, ist.

14. Zusammensetzung gemäß einem der Ansprüche 12 oder 13, **dadurch gekennzeichnet, dass** das Polymer in der festen Phase ist und 1 bis 5 Gew.%, bezogen auf das Gesamtgewicht der festen Phase, vorteilhafterweise 2 bis 4% ausmacht.

15. Zusammensetzung gemäß einem der Ansprüche 12 oder 13, **dadurch gekennzeichnet, dass** das Polymer in der flüssigen Phase ist und 0,2 bis 10 Gew.%, bezogen auf das Gesamtvolumen der flüssigen Phase, ausmacht.

16. Verfahren zur Herstellung eines Zements aus einer Zusammensetzung gemäß einem der Ansprüche 4 bis 15, **dadurch gekennzeichnet, dass** es wenigstens eine Stufe umfasst, in der man die feste Phase mit der flüssigen Phase mischt und in der die Reaktion:
6Ca₍₁₋ₓ₎Sr₍ₓ₎HPO₄ + yCaO → Ca₍₁₀₋₆ₓ₎Sr₆ₓ(PO₄)₆(OH)₂ + (y-4)Ca(OH)₂
erfolgt.

17. Dentalkit, umfassend eine Zusammensetzung gemäß einem der Ansprüche 4 bis 15 in Form einer flüssigen Phase und einer festen Phase, die in getrennten Kompartimenten aufbewahrt sind.

18. Verwendung einer Zusammensetzung gemäß einem der Ansprüche 4 bis 15 zur Herstellung eines endodontischen Zements, der für eine der folgenden Anwendungen bestimmt ist:
- Obturation der Zahnwurzelkanäle,
- Pulpa-Überkappung,
- Reparatur einer Perforation,
- Bildung eines apikalen Pfropfens.

## Claims

1. A compound according to formula (I) below:
Ca₍₁₋ₓ)Sr₍ₓ₎HPO₄ (I)
in which x represents a number between 0.45 and 0.55.

2. A process for the production of a compound of formula (I):
Ca₍₁₋ₓ)Sr₍ₓ₎HPO₄ (I)
in which x represents a number between 0.2 and 0.8, **characterised in that** it comprises a step of mixing calcium bis-dihydrogen phosphate monohydrate and strontium hydroxide octahydrate in powder form according to one of the reactions:
For 0.5 ≤ x ≤ 0.8
(1-x)Ca(H₂PO₄)₂, H₂O + (2x-1)H₃PO₄ + x Sr(OH)₂, 8H₂O → Ca₍₁₋ₓ₎ SrₓHPO₄ + (9x + 1)H₂O
For 0.2 ≤ x ≤ 0.5
Ca(H₂PO₄)₂,H₂O + 2x Sr(OH)₂, 8H₂O + (1-2x) Ca(OH)₂ → 2 Ca₍₁₋ₓ₎SrₓHPO4 +(16x + 3)H₂O

3. A process according to claim 2, **characterised in that** mixing is carried out in a ball mill or through simple mixing in a mortar in the presence of a small quantity of water, and possibly in the presence of orthophosphoric acid or calcium hydroxide, at ambient temperature, followed by drying.

4. A composition comprising a solid phase (SP) and a liquid phase (LP), the solid phase comprising a mixture of minerals of formula (II):
6 Ca₍₁₋ₓ)Sr₍ₓ₎HPO₄ + y CaO + zA (II)
in which:
x represents a number of between 0.2 and 0.8;
y represents a number of between 5 and 12;
A represents a radio-opaque compound;
z represents a number of between 1 and 3, and
the liquid phase comprises water or an aqueous salt solution of pH between 5 and 9,
for use as an endodontic cement.

5. A composition according to claim 4, in which x represents a number of between 0.3 and 0.7, more preferably between 0.4 and 0.6, and advantageously between 0.45 and 0.55.

6. A composition according to claim 4 or 5, in which A is selected from a salt of bismuth, barium or strontium, a compound selected from: a carbonate, sulfate, fluoride or phosphate.

7. A composition according to any one of claims 4 to 6, **characterised in that** y is taken to be between 7 and 12, and z lies between 1.5 and 2.5.

8. A composition according to any one of claims 4 to 7, **characterised in that** y is taken to be between 8 and 10.

9. A composition according to any one of claims 4 to 6, **characterised in that** y is taken to be between 5 and 7.

10. A composition according to any one of claims 4 to 9, **characterised in that** the liquid phase is selected from:
- an aqueous solution comprising 0.2 to 1.5 mol/l of a mixture of NaH₂PO₄ and Na₂HPO₄,
- an aqueous solution comprising 0.2 to 1.5 mol/l of a mixture of NaH₂PO₄ and sodium glycerophosfate hexahydrate,
- an aqueous solution comprising 0.2 to 1.5 mol/l of a mixture of NH₄H₂PO₄ and (NH₄)₂HPO₄,
- an aqueous solution comprising 0.2 to 1.5 mol/l of trisodium citrate.

11. A composition according to any one of claims 4 to 10, **characterised in that** the ratio between the volume of the liquid phase (L) and the weight of the solid phase (P) is 0.2 < L/P < 0.7 ml/g, advantageously 0.3 < L/P < 0.6 ml/g.

12. A composition according to any one of claims 4 to 11, **characterised in that** it further comprises at least one polymer selected from the polyacrylates and the alginates.

13. A composition according to claim 12, **characterised in that** the polymer is a polyacrylate of molecular weight between 2000 and 50,000, advantageously between 3000 and 30,000, and even more advantageously between 5000 and 20,000.

14. A composition according to either of claims 12 or 13, **characterised in that** the polymer is in the solid phase and represents 1 to 5% by weight in relation to the total weight of the solid phase, advantageously from 2 to 4%.

15. A composition according to either of claims 12 or 13, **characterised in that** the polymer is in the liquid phase and represents from 0.2 to 10% by weight in relation to the total volume of the liquid phase.

16. A process for preparing a cement from a composition according to any one of claims 4 to 15, **characterised in that** it comprises at least one stage in which the solid phase is mixed with the liquid phase and in which the reaction:
6 Ca₍₁₋ₓ₎SrₓHPO₄ + y CaO → Ca₍₁₀₋₆ₓ₎Sr₆ₓ(PO₄)₆(OH)₂ + (y-4) Ca(OH)₂ takes place.

17. A dental kit comprising a composition according to any one of claims 4 to 15 in the form of a liquid phase and a solid phase kept in separate compartments.

18. Use of a composition according to any one of claims 4 to 15 for the preparation of an endodontic cement intended for one of the following applications:
- the filling of tooth root canals,
- the preparation of crowns,
- the filling of cavities,
- the formation of apical plugs.
